Europäisches Patentamt
European Patent Office
Office européen des brevets

(19)

(11) Publication number: **0 115 690**
**A1**

(12) **EUROPEAN PATENT APPLICATION**

(21) Application number: **83307839.7**

(22) Date of filing: **21.12.83**

(51) Int. Cl.³: **C 07 C 57/62**, C 07 C 59/72, C 07 C 69/618, A 61 K 31/19, A 61 K 31/215, A 61 K 31/41, C 07 D 257/04

(30) Priority: **03.01.83 US 454941**

(43) Date of publication of application: **15.08.84 Bulletin 84/33**

(84) Designated Contracting States: **AT BE CH DE FR IT LI LU NL SE**

(71) Applicant: **AMERICAN HOME PRODUCTS CORPORATION, 685, Third Avenue, New York, New York 10017 (US)**

(72) Inventor: **Wolf, Milton, 1502 Wilson Lane, West Chester Pennsylvania (US)**

(74) Representative: **Porter, Graham Ronald et al, C/O John Wyeth & Brother Limited Huntercombe Lane South Taplow, Maidenhead Berkshire, SL6 0PH (GB)**

(54) **Dibenzocycloheptenylidenes.**

(57) Compounds having the formula:

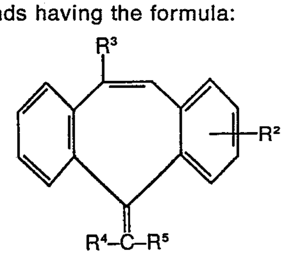

B

wherein $R^4$ is hydrogen or chloro;
$R^5$ is carboxy, an ester thereof, or tetrazolyl;
$R^2$ is hydrogen, halo, or alkoxy of 1 to 4 carbon atoms; and
$R^3$ is hydrogen or bromo,
with the proviso that when $R^4$ is hydrogen, $R^5$ is carboxy and $R^2$ or $R^3$ is other than hydrogen; and that when $R^4$, $R^2$ and $R^3$ are all hydrogen, $R^5$ is tetrazolyl; the stereoisomers thereof and the pharmaceutically acceptable salts thereof. The compounds of formula B wherein $R^5$ is carboxy or tetrazolyl have pharmaceutical utility eg. as anti-inflammatory agents. The compounds where $R^5$ is a carboxylic ester are intermediates for the corresponding acids. Pharmaceutical composition containing the active compounds are also disclosed.

EP 0 115 690 A1

This invention relates to certain dibenzocycloheptenylidenes to pharmaceutical compositions containing them and to the use of such dibenzocycloheptenylidenes in treating inflammation in mammals.

In the treatment of chronic inflammatory conditions, both steroidal and nonsteroidal drugs have been extensively used. The use of corticosteroid therapy in rheumatoid arthritis for example, while providing symptomatic relief, has not been shown actually to improve prognosis and prolonged therapy is attended by serious side effects, such as increased susceptibility to infection, peptic ulceration and development of osteoporosis.

The non-steroidal anti-inflammatory agents, such as the salicylates, indomethacin, phenylbutazone, gold salts and the like, are also used in the treatment of chronic inflammatory diseases, especially rheumatoid arthritis. The non-steroidal drugs have a suppressive effect on the antigen-antibody reactions which are causative factors in the inflammatory process, and also have effects on the synthesis or release of prostaglandins, the complement system, lymphocyte transformations and so forth. However, as with the steroids, these anti-inflammatory agents, when used steadily to control chronic inflammatory conditions, have serious side-effects. The most serious is gastric intolerance, which severely limits the use of these agents in peptic ulcer patients. Moreover, phenylbutazone can cause bone marrow depression. Thus, the most widely used non-steroidal anti-inflammatory agents have some serious side-effects which limit their usefulness in the treatment of chronic inflammatory conditions.

In our GB Patent 2071098 we have described pharmaceutical compositions containing dibenzocycloheptenylidene compounds of formula A

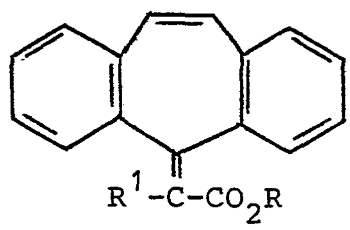

A

Where R is hydrogen or an alkyl group of 1-4 carbon atoms, $R^1$ is hydrogen or an alkyl group of 1-4 carbon atoms or pharmaceutically acceptable salts thereof and a pharmaceutically acceptable carrier. The compounds of formula A have anti-flammatory activity and are useful in the treatment of anti-flammatory conditions, especially chronic conditions with little or no secondary effects.

We have now found that by modifying the molecule of compounds of formula A further anti-inflammatory agents are obtained which have considerable value in the treatment of inflammatory conditions, expecially chronic conditions, with little or no secondary effects.

The invention is directed to compounds of the formula:

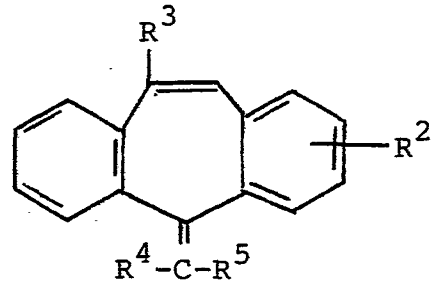

B

wherein $R^4$ is hydrogen or chloro, $R^5$ is carboxy, an ester thereof or tetrazolyl, $R^2$ is hydrogen, halo or alkoxy of 1-4 carbon atoms, and $R^3$ is hydrogen or bromo, with the proviso that when $R^4$ is hydrogen and $R^5$ is carboxy then $R^2$ or $R^3$ is other than hydrogen; and that when $R^4$, $R^2$ and $R^3$ are all hydrogen, $R^5$ is tetrazolyl; the stereoisomers thereof and the pharmaceutically acceptable salts thereof.

The invention is also directed to the use of compounds of formula B wherein $R^5$ is carboxy or tetrazolyl as pharmaceuticals eg. as anti-flammatory agents.

The halo group $R^2$ is preferably fluoro, chloro or bromo. When $R^4$ is chloro preferably $R^5$ is carboxy and $R^2$ and $R^3$ are hydrogen.

The compounds of formula B can be prepared by various methods all of which are included in the invention.

One method comprises treating a compound of formula D

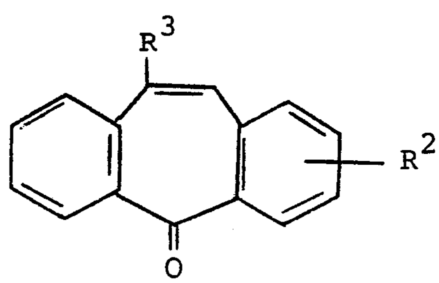

D

wherein $R^2$ and $R^3$ are as defined above in known manner to introduce the group $R^4-\overset{\|}{C}-R^5$ wherein $R^4$ and $R^5$ are as defined in connection with formula B and if desired converting the product to a pharmaceutically acceptable salt.

Compounds of Formula B wherein $R^5$ is carboxy may be prepared by hydrolysis of the corresponding carboxylic esters.

Compounds of Formula B wherein $R^5$ is tetrazolyl may be prepared by a process in which a compound of Formula C

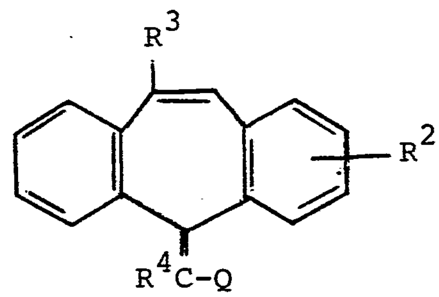

C

wherein $R^2$, $R^3$ and $R^4$ are as defined above and Q is a group containing an unsaturated -C-N linkage, is reacted with other known compounds to form a tetrazole ring system and thus a product B wherein $R^5$ is tetrazolyl and if desired the product is converted to a pharmaceutically acceptable salt.

Thus a compound of Formula C wherein Q is $-C{\equiv}N$ or $\underset{\underset{G}{|}}{C}{=}NH$ wherein G is a halogen atom or an alkoxy group is reacted with an alkali metal azide or ammonium azide to obtain a compound of formula B wherein $R^5$ is tetrazolyl and if desired the product is converted to a pharmaceutically acceptable salt.

The compounds of formula B can be prepared according to the following reaction sequence:

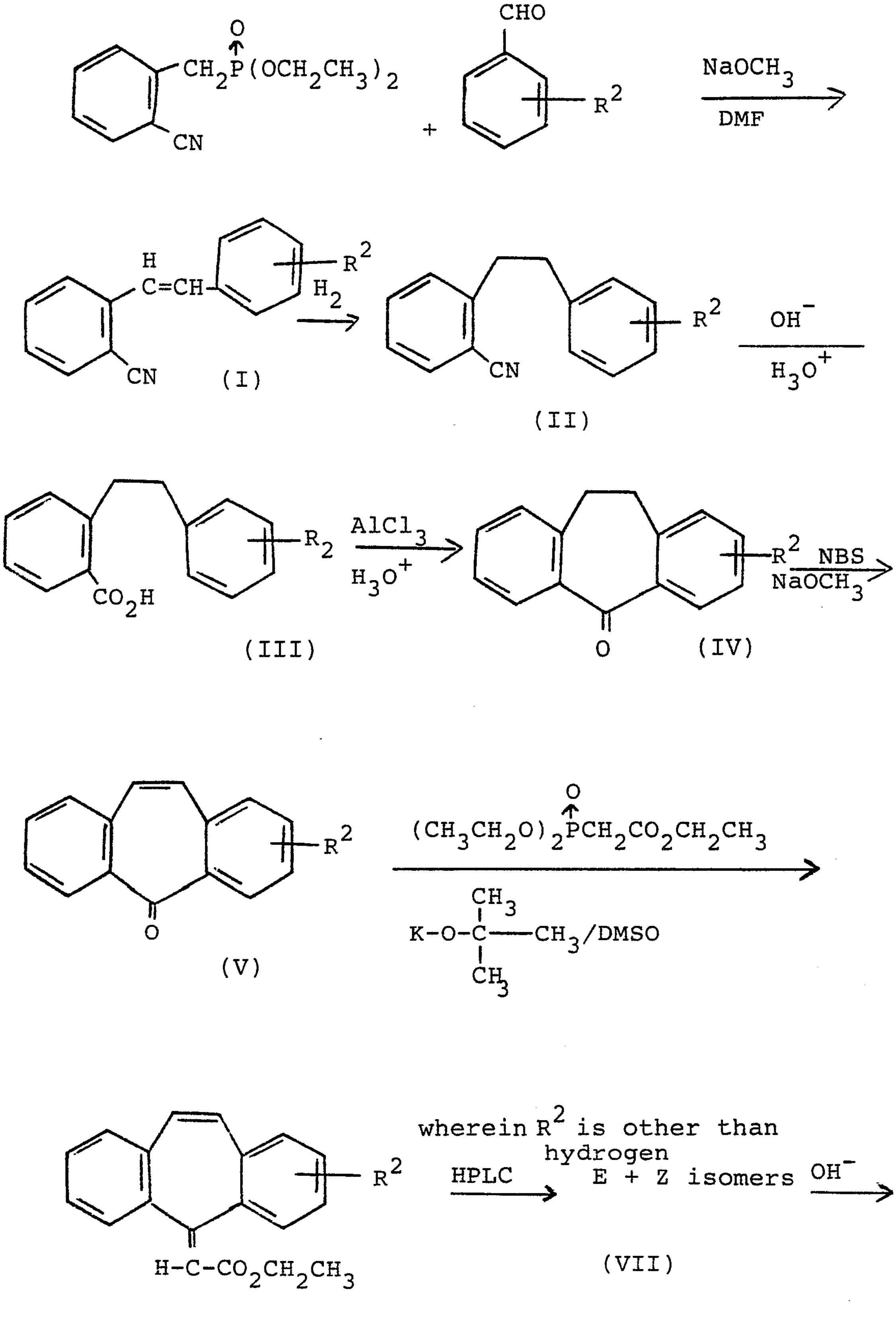


(I) (II) (III) (IV) (V) (VI) (VII)

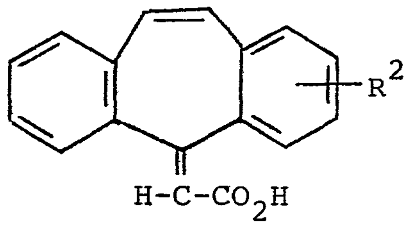

and

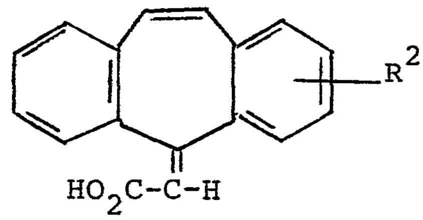

(Z-isomer) (E-isomer)

(VIII)

In this sequence, diethyl[(2-cyanophenyl)methyl]phosphonate is reacted with a suitably substituted benzaldehyde in the presence of a metal alkoxide, such as sodium methoxide in a solvent such as dimethylformamide. The resultant intermediate (I) is reduced to yield intermediate (II). This benzonitrile is hydrolyzed to the corresponding carboxylic acid (III), which is then subjected to ring closure to yield the intermediate 10,11-dihydro-dibenzocyclohepten-5-one (IV). The latter is treated with N-bromosuccinimide in carbon tetrachloride followed by treatment with a metal alkoxide in organic solvent to yield the dibenzocyclohepten-5-one (V). The intermediate is reacted with a trialkyl phosphonoalkanoate and an alkali metal alkoxide eg. potassium t-butoxide in an organic solvent, to yield the esters (VI) of the desired acids(, a mixture of E- and Z-isomers when $R^2$ is other than hydrogen).

The racemic mixture is separated using high pressure liquid chromatography (HPLC) to yield the separate E-and Z-isomers (VII), which may then be saponified with a base and the resultant final products (VIII) recovered as salts or as the free acids. Alternatively, the intermediate (V) can be subjected to a Reformatsky reaction or a modified Reformatsky reaction using lithium salts of α-lithiocarboxylic acids to yield the intermediate (VI).

The compounds in which $R^4$ is chloro and $R^5$ and $R^2$ are both hydrogen can be prepared by a variation of the above-described reaction sequence. The intermediate (IV) in which $R^2$ is hydrogen, is reacted with a trialkyl phosphonoalkanoate and a metal alkoxide, such as potassium t-butoxide to yield an alkyl-10,11-dihydro-dibenzocyclohepten-5-ylidene acetate. The latter is reacted with chlorine in carbon tetrachloride to yield the 5,α-dichloro derivative, which is treated with a metal alkoxide, such as potassium t-butoxide in tetrahydrofuran to yield the α-chloro derivative. The latter is then treated with N-bromosuccinimide in carbon tetrachloride followed by treatment with triethylamine to yield an alkyl α-chloro-dibenzocyclohepten-5-ylidene acetate, which may then be saponified with a base and the desired final product α-chloro derivative is recovered as a salt or as the free acid.

When it is desired to prepare the compound in which $R^5$ is tetrazolyl, the intermediate (V) in which $R^2$ is hydrogen, may be reacted with a dialkyl cyanomethyl phosphonate and a metal alkoxide, such as potassium t-butoxide, to yield dibenzocyclohepten-5-ylidene acetonitrile, which is reacted with sodium azide and ammonium chloride in organic solvent to yield the desired dibenzocyclohepten-5-ylidenemethyl tetrazole.

The compound 5H-dibenzo[a,d]cyclohepten-5-one, which is used as a starting material in some of the outlined reaction sequences, can be prepared according to the method of W. Treibs and H.J. Klinkhammer, Chem. Ber., 84, 671 (1951) or it can be obtained commercially.

The compounds of the invention in which the $R^2$ substituent is other than hydrogen exist as conformational isomers. As indicated supra, the racemic mixture of the E- and Z-isomers may be separated into the

individual component isomers by use of high pressure liquid chromatography. While in all cases the racemic mixtures of formula B wherein $R^5$ is carboxy or tetrazolyl exhibit anti-inflammatory activity, generally the E-isomer exhibits greater activity than Z-isomer. For example, the E-isomer of (3-chloro-5H-dibenzo[a,d]cyclohepten-5-ylidene)acetic acid is several times more active than its Z-isomer. Accordingly, in some of the compounds of the invention, the unresolved racemic mixtures and individual isomers are both active anti-inflammatory agents.

The pharmaceutically acceptable salts of the free acid wherein $R^5$ is carboxy or tetrazolyl include the sodium, potassium, ammonium, and lower alkylamine salts, which are prepared and isolated by conventional methods.

The compounds of formula B wherein $R^5$ is carboxy or tetrazolyl are anti-inflammatory agents having significant oral activity in the treatment of inflammation. These compounds do not act by stimulating the steroids naturally occurring in mammals, and since these compounds additionally have a lack of general pharmacological activity, they are of particular use in the long-term treatment of chronic inflammatory conditions, which, for example, are present in rheumatoid arthritis and other connective tissue disorders.

When the compounds of the invention are employed as anti-inflammatory agents, they can be formulated into oral dosage forms such as tablets, capsules and the like. The compounds can be administered alone or by combining them with conventional carriers, such as magnesium carbonate, magnesium stearate, talc, sugar, lactose, pectin, dextrin, starch, gelatin, tragacanth, methyl cellulose, sodium carboxymethyl cellulose, low melting wax, cocoa butter, and the like. Diluents, flavouring agents, solubilizers,

lubricants, suspending agents, binders, tablet-disintegrating agents and the like may be employed. The compound may be encapsulated with or without other carriers. In all cases the proportion of active ingredients in said compositions both solid and liquid will be at least sufficient to impart anti-inflammatory activity thereto on oral administration. The compounds may also be injected parenterally, in which case they are used in the form of a sterile solution containing other solutes, for example, enough saline or glucose to make the solution isotonic.

The dosage requirements vary with the particular compositions employed, the route of administration, the severity of the symptoms presented and the particular subject being treated. Treatment will generally be initiated with small dosages less than the optimum dose of the compound. Thereafter, the dosage is increased until the optimum effect under the circumstances is reached. In general, the compounds of the invention are most desirably administered at a concentration level that will generally afford effective results without causing any harmful or deleterious side effects. With large animals (about 70kg. body weight), for oral administration the dose is from about 10 milligrams to about 300 milligrams and preferably from about 10 milligrams to about 200 milligrams per day either as a single unit dose, or if desired, the dosage may be divided into convenient subunits administered at specified times throughout the day.

The anti-inflammatory effects of the compounds of the invention may be demonstrated by standard pharmacological procedures, which are described more fully in the examples given hereinafter. These procedures illustrate the ability of the compounds of the invention to exert an anti-flammatory effect by directly measuring

the effect of the compounds in the rat carrageenan edema test, and the rat adjuvant arthritis test.

## Example 1

(3-Chloro-5H-dibenzo[a,d]cyclohepten-5-ylidene) acetic acid

A. E-2-[2-(4-chlorophenyl)ethenyl]benzonitrile

A solution of diethyl [(2-cyanophenyl)methyl]phosphonate (75.7g, 0.30m) and p-chlorobenzaldehyde (43.5g, 0.30m of 97% in dry dimethylformamide (225ml) is added dropwise with stirring to a magnetically stirred suspension of sodium methoxide (24.3g) in dimethylformamide (150ml) in a nitrogen atmosphere over a period of about two hours. During the addition, the pot temperature is maintained at -5° to +5°C. (ice/salt bath). After the addition is complete, the mixture is stirred at 0 to 5°C for one hour, then allowed to warm to ambient temperature and stirred overnight. The mixture is poured into water (4L) with stirring. After stirring for two hours the off-white crystalline solid is collected and dried at 65°C/O. 1mm overnight. The yield of the title compound melting at 81.0-83.5°C is 70.1g (98.9%).

B. 2-[2-(4-Chlorophenyl)ethyl]benzonitrile

Sodium borohydride (0.19g) is added to a magnetically stirred suspension of palladium chloride (0.443g) in 1,2- dimethoxyethane (40ml). After stirring at ambient temperature for one-half hour, a solution of 2-[-(4-chlorophenyl)ethenyl]-benzonitrile (60.0g., 0.2503m) in 1,2-dimethoxyethane is added. The mixture is hydrogenated in a Parr apparatus. The theoretical amount of hydrogen is taken up in about forty-five minutes. The solution is filtered through Celite to

remove the palladium affording a clear, almost colourless filtrate. Evaporation of the solvent in vacuo affords a white solid melting at 64-68°C (uncorr.) in a yield of 60.4g (99.8%). Recrystallisation from methylene chloride-pentane affords colourless prisms melting at 67.0-68.5°C (uncorr.).

C. 2-[2-(4-Chlorophenyl)ethyl]benzoic acid

2-[-(4-Chlorophenyl)ethyl]benzonitrile (60.3g., 0.250m) is added to a solution of potassium hydroxide (66.0g of 85%, 1.0mmol) in distilled water (110ml). The mixture is refluxed for fifty and one-half hours. The cooled solution is poured into water (1.5L) and the small amount of insoluble material separated by filtration. The clear filtrate is added in a thin stream with stirring to 6N hydrochloric acid (ca. 200ml). The product separates as colourless crystals which are collected and dried at 80°C/20mm Hg. The yield of title compound melting at 125.0-127.0°(uncorr.) is 50.4g (77.3%).

D. 3-Chloro-10,11-dihydro-5H-dibenzo[a,d]cyclohepten-5-one

A mixture of oxalyl chloride (24.1g. 0.190m) and 2-[2-(4-chlorophenyl)ethyl]benzoic acid (45.0g, 0.173m) in dry methylene chloride is stirred at ambient temperature until gas evolution ceases (ca one hour). The mixture is refluxed for one hour. The solvent is evaporated in vacuo affording an amber oily residue which crystallises on cooling. The crystalline acid chloride is dissolved in methylene chloride (150ml) and added dropwise to a magnetically stirred suspension of anhydrous aluminum chloride (24.3g., 0.190m) in methylene chloride (250ml) over a period of ca one-half hour.

The dark homogeneous solution is stirred at ambient temperature for twenty-one hours, then refluxed until hydrogen chloride evolution almost ceases. The dark green mixture is poured into 2N-hydrochloric acid (1.2L). The organic layer is separated and successfully washed with 2N hydrochloric acid (2 x 250ml), saturated sodium bicarbonate solution (2 x 250ml), distilled water (250ml), brine (250ml) then filtered through anhydrous sodium sulphate. The amber filtrate is concentrated in vacuo to give a viscous oil (39.6g. 94.3%). The oil is distilled in vacuo to give a yellow oil boiling at 176.5-179.0°C. at 0.9-1.0mm in a yield of 26.0g (61.9%). Addition of a seed of authentic title compound produces crystallisation.

| | | | |
|---|---|---|---|
| Analysis for: | $C_{15}H_{11}ClO$ | | |
| Calculated: | C,74.23; | H,4.57; | Cl,14.61. |
| Found: | C,74.12; | H,4.69; | Cl,14.16 |

E. 3-Chloro-5H-dibenzo[a,d]cyclohepten-5-one

A mixture of 3-chloro-10,11-dihydro-5H-dibenzo[a,d]-cyclohepten-5-one (25.8g, 0.106m), N-bromosuccinimide (19.1g, 0.106m) and 2,2'-azobis[2-methylpropronitrile] (1.0g) in carbon tetrachloride (400ml) is heated gently until a vigorous reaction begins. The heat source is removed. After the initial reaction subsides, the mixture is refluxed for one hour, then allowed to cool to ambient temperature. The succinimide is separated by filtration, washed with carbon tetrachloride, dried at 80°C/20mm Hg. The yield of succinimide melting at 122.0-123.5°C (uncorr.) is 10.4g (98.6%). The filtrate is concentrated in vacuo to give a red oily residue to which is added methanol (300ml) and sodium methoxide (10.8g. 0.20m). After the initial vigorous reaction, the magnetically stirred mixture is refluxed

for one hour and fifteen minutes. After the solvent is evaporated *in vacuo*, the oily residue is diluted with water (250ml) and extracted with methylene chloride (3 x 250ml). The combined extracts are successively washed with distilled water (2 x 250ml), brine (250ml), filtered through anhydrous sodium sulphate. The filtrate is evaporated *in vacuo* to give a tan solid melting at 78-86°C (uncorr.) in a yield of 26.5g (>100%, solvated). The crude product is subjected to preparative HPLC (Waters Silica Gel column, 90% hexane-10% methylene chloride) to give the pure title compound.

F. Ethyl-(3-Chloro-5*H*-dibenzo[*a*,*d*]cyclohepten-5-ylidene)acetate

A solution of triethyl phosphonoacetate (18.384g, 0.0781m) in dry dimethyl sulphoxide (25ml) is added dropwise to a magnetically stirred solution of potassium t-butoxide (9202g, 0.0820m) in dry dimethyl sulphoxide (100ml) in a nitrogen atmosphere over a period of *ca* fifteen minutes. After stirring at ambient temperature for fifteen minutes, a solution of 3-chloro-5*H*-dibenzo[*a*,*d*]cyclohepten-5-one (18.8g, 0.078m) in dry dimethyl sulphoxide (150ml) is added in a single portion. The dark mixture is then heated at 100±1°C for twenty four hours. The solvent is evaporated *in vacuo* to give a partially solidified residue which is diluted with water (150ml). This mixture is extracted with methylene chloride (1 x 150ml + 3 x 100ml). The combined extracts are successively washed with distilled water (3 x 100ml), brine (100ml) and filtered through anhydrous sodium sulphate. The clear amber filtrate is concentrated *in vacuo* to give an amber oil (24.9g) which is subjected to HPLC separation (Waters Silica Gel column, hexane-ethylacetate,

99:1) of the E- and Z-isomers.

The first (faster moving) isomer fractions to come off the column (fractions 14, 15 and 16) are combined and evaporated in vacuo to give a pale yellow oil (6.9g) which crystallises on cooling. This material melts at 56.0-57.0°C (uncorr.). Recrystallisation of this material from pentane affords colourless prisms melting at 58.5-60.0°C. (uncorr.). This material is shown to be the Z-isomer by NMR analysis with $Eu(fod)_3$ shift reagent.

| | | | |
|---|---|---|---|
| Analysis for: | $C_{19}H_{15}ClO_2$ | | |
| Calculated: | C,73.43; | H,4.87; | Cl,11.41 |
| Found: | C,73.15; | H,4.95; | Cl,11.33 |

The slower moving isomer comes off the column in fractions 19-22. These are combined and evaporated in vacuo to give a colourless solid (6.4g) melting at 91.5-92.5°C.(uncorr). A sample recrystallised from cyclohexane gives a colourless needles melting at 92.0-92.5°C.(uncorr.). This material is shown to be the E-isomer by NMR analysis using $Eu(fod)_3$ shift reagent.

| | | | |
|---|---|---|---|
| Analysis for: | $C_{19}H_{15}ClO_2$ | | |
| Calculated: | C,73.43; | H,4.87; | Cl,11.41 |
| Found: | C,73.27; | H,5.03; | Cl,11.78 |

G. Z-(3-Chloro-5H-dibenzo[a,d]cyclohepten-5-ylidene) acetic acid

A mixture of Z-ethyl(3-chloro-5H-dibenzo[a,d]cyclohepten-5-ylidene) acetic acid (5.65g. 0.0182m),ethanol (100ml) and a solution of potassium hydroxide (2.4g of 85%, 0.036m) in distilled water (50ml) is refluxed for twenty-three hours. The ethanol is evaporated in vacuo,

the residue diluted with distilled water (50ml), filtered to remove a trace of solid, then added dropwise with stirring to 2.5N hydrochloric acid (25ml). The product separates as a colourless solid which is collected, washed with distilled water, dried at 78°C/0.1mm overnight. The yield of colourless crystals melting at 254.5°C dec. (uncorr) is 4.907g (95.4%). Recrystallisation of this material from tetrahydrofuran-cyclohexane affords colourless needles melting at 261.0°C dec (uncorr.) in a yield of 3.633g (70.6%).

| | | | |
|---|---|---|---|
| Analysis for: | $C_{17}H_{11}ClO_2$ | | |
| Calculated: | C,72.22; | H,3.92; | Cl,12.52 |
| Found: | C,72.04; | H,4.09; | Cl,12.34 |

H. E-(3-Chloro-5H-dibenzo[a,d]cyclohepten-5-ylidene acetic acid

A mixture of E-ethyl-(3-chloro-5H-dibenzo[a,d]cyclohepten-5-ylidene) acetate (5.70g, 0.183m), ethanol (100ml) and a solution of potassium hydroxide (2.4g of 85%, 0.036m) in distilled water (50ml) is refluxed overnight (eighteen hours). The ethanol is evaporated *in vacuo*, the oily residue diluted with distilled water (50ml), then added dropwise with stirring to 2.5N hydrochloric acid (25ml). The product separates as a colourless solid which is collected, washed with distilled water, dried at 78°C/0.1mm overnight. The yield of crude product melting at 220.0-222.0°C (uncorr.) is 5.069g (97.9%). Recrystallisation of this material from tetrahydrofuran-cyclohexane affords colourless plates melting at 221.5°C dec. (uncorr.) in a yield of 3.498g (67.62%).

| | | | |
|---|---|---|---|
| Analysis for: | $C_{17}H_{11}ClO_2$ | | |
| Calculated: | C,72.22; | H,3.92; | Cl,12.54 |
| Found: | C,72.02; | H,4.18; | Cl,12.24 |

## Example 2

**(2-Chloro-5H-dibenzo[a,d]cyclohepten-5-ylidene acetic acid**

A. E-2-[2-(3-Chlorophenyl)ethenyl]benzonitrile

The title compound is prepared in a manner similar to that of Example 1A. Recrystallisation of the crude product from ether-hexane affords crystals melting at 97.5-98.5°C (uncorr.) in a yield of 83.0%.

| | | | | |
|---|---|---|---|---|
| Analysis for: | $C_{15}H_{10}NCl$ | | | |
| Calculated: | C,75.16; | H,4.21; | N,5.84; | Cl,14.79 |
| Found: | C,74.68; | H,4.33; | N,5.80; | Cl,14.46 |

B. E-2-[2-(3-Chlorophenyl)ethyl]benzonitrile

The title compound is prepared in a manner similar to that of Example 1B. The title compound is obtained in quantitative yield (crude). A portion recrystallised from chloroform-hexane and finally from cyclohexane melts at 54.0-56.0°C (uncorr.).

| | | | | |
|---|---|---|---|---|
| Analysis for: | $C_{15}H_{12}NCl$ | | | |
| Calculated: | C,74.53; | H,5.01; | N,5.80; | Cl,14.67 |
| Found: | C,74.49; | H,5.15; | N,5.75; | Cl,14.37 |

C. 2-[2-(3-Chlorophenyl)ethyl]benzoic acid

The title compound is prepared in a manner similar to that of Example 1C. The crude acid melting at 89-90°C (uncorr.) is obtained in 79.0% yield. The product is characterised by IR and NMR spectra.

D. 2-[2-Chloro-10,11-dihydro-5H-dibenzo[a,d]cyclohepten-5-one

Phosphorus pentachloride (22.821g, 0.107m) is added portionwise to a solution of 2-[2-(3-chlorophenyl)ethyl] benzoic acid (26.56g, 0.102m) in methylene chloride (250ml) in a nitrogen atmosphere. After the addition is complete, the mixture is refluxed for two and one-half hours, then concentrated *in vacuo* to give an oil. Toluene (50ml) is added to the oil, and the solution concentrated *in vacuo*. This is repeated twice to ensure removal of phosphorus oxychloride. The acid chloride, 2-[2-chlorophenyl)-ethyl]benzoyl chloride, melting at 53.0-56°C (uncorr.) is obtained in a 30.0g yield. The acid chloride (30.0g,m.) in methylene chloride (100ml) is added dropwise with stirring in a nitrogen atmosphere to a suspension of aluminium chloride (14.67g, 0.11m) in methylene chloride (300ml). After the addition is complete, the mixture is stirred overnight at ambient temperature then refluxed for one hour. The mixture is chilled in an ice bath, then hydrolysed by dropwise addition of 6N hydrochloric acid (225ml) with stirring. The mixture is evaporated *in vacuo*, and the aqueous residue extracted with ether (4 x 200ml). The combined extracts are successively washed with water (3 x 150ml), 5% sodium bicarbonate solution (2 x 125ml), water (2 x 200ml), brine (500ml) and filtered through anhydrous sodium sulphate. The clear filtrate is concentrated *in vacuo* affording the crude title compound in quantitative yield, mp 66-72°C (uncorr.).

E. 2-Chloro-5*H*-dibenzo[*a*,*d*]cyclohepten-5-one

The title compound is prepared from the compound of D above in a manner similar to that of Example 1E. The crude product, melting at 157.5-158.5°C (uncorr.) is obtained in 91.0% yield. The structure is confirmed by NMR spectrum.

F. Ethyl-(2-chloro-5H-dibenzo[a,d]cyclohepten-5-ylidene)acetate

The title compound is prepared from the compound of E above and separated into Z- and E-isomers as described in Example 1F. The Z-isomer melts at 106-107°C (uncorr.) and the E-isomer melts at 81.5-82.5°C (uncorr).

G. Z-(2-Chloro-5H-dibenzo[a,d]cyclohepten-5-ylidene acetic acid

The title compound is prepared from the Z-isomer obtained in Step F above, in a manner similar to that of Example 1G. The crude acid is recrystallised from cyclohexane and is obtained as colourless crystals melting at 205.5-206.5°C (uncorr).

Analysis for: $C_{17}H_{11}ClO_2$

Calculated: C, 72.22; H, 3.92; Cl, 12.54

Found: C, 72.30; H, 4.12; Cl, 12.53

H. E-(2-Chloro-5H-dibenzo[a,d]cyclohepten-5-ylidene acetic acid

The title compound is prepared in a manner similar to that of Example 1H. The crude acid is recrystallised from ether-hexane affording tan crystals melting at 96.5-97.5°C (uncorr.)

Analysis for: $C_{17}H_{11}ClO_2$

Calculated: C, 72.22; H, 3.92; Cl, 12.54

Found: C, 72.19; H, 3.99; Cl, 12.67

Example 3

(1-Chloro-5H-dibenzo[a,d]cyclohepten-5-ylidene)acetic acid

A. E-2-[2(2-Chlorophenyl)ethenyl]benzonitrile

The title compound is prepared from diethyl[(2-cyanophenyl)methyl]phosphonate and 2-chlorobenzaldehyde in a manner similar to that of Example 1A. The crystalline product melting at 119.0-120.0°C (uncorr.) is obtained in 74% yield.

Analysis for: $C_{15}H_{10}ClN$

Calculated: C, 75.16; H, 4.21; Cl, 14.75; N, 5.84

Found: C, 74.91; H, 4.39; Cl, 14.46; N, 5.74

B. 2-[2-(2-Chlorophenyl)ethyl]benzonitrile

The title compound is prepared from E-2-[2-(2-chlorophenyl)ethenyl]benzonitrile in quantitative yield in a manner similar to that of Example 1B. The reduced nitrile melts at 59.0-61.0°C. (uncorr.)

Analysis for: $C_{15}H_{12}ClN$

Calculated: C,74.53; H, 5.01; N, 5.80; Cl, 14.67

Found: C,74.57; H, 5.11; N, 5.74; Cl, 14.35

C. 2-[2-(2-Chlorophenyl)ethyl]benzoic acid

The title compound is obtained from 2-[2-(2-chlorophenyl)ethyl]benzonitrile, as a crystalline compound melting at 135.0-137.5°C (uncorr.), in a manner similar to that of Example 1C.

Analysis for: $C_{15}H_{13}ClO_2$

Calculated: C, 69.01; H, 5.03; Cl, 13.60

Found: C, 69.13; H, 5.02; Cl, 13.29.

D. 1-Chloro-10,11-dihydro-5H-dibenzo[a,d]cyclohepten 5-one

Treatment of 2-[2-(2-chlorophenyl)ethyl]benzoic acid in a manner similar to that of Example 1D affords the title compound as an oil in quantitative yield. Chromatography on silica gel gives the purified title compound in 74% yield.

E. 1-Chloro-5H-dibenzo[a,d]cyclohepten-5-one

Bromination and dehydrobromination of 1-chloro-10,11-dihydro-5H-dibenzo[a,d]cyclohepten-5-one in a manner similar to that of Example 1E affords the title compound as colourless crystals melting at 135.0-136.5°C (uncorr.) in 26% yield.

F. (1-Chloro-5H-dibenzo[a,d]cyclohepten-5-ylidene acetic acid

Horner-Wittig condensation of 1-chloro-5H-dibenzo[a,d]-cyclohepten-5-one in a manner similar to that of Example 1F., affords a mixture of E- and Z-isomers of ethyl (1-chloro-5H-dibenzo-[a,d]cyclohepten-5-ylidene)acetate in 97% yield. Hydrolysis of the mixture of isomeric esters as in Example 1G, gives the title compound as a mixture of E- and Z-isomers melting at 199-214°C (uncorr.) in 78% yield.

Analysis for: $C_{17}H_{11}ClO_2$

Calculated: C, 72.22; H, 3.92; Cl, 12.54

Found: C, 71,82; H, 4.23; Cl, 12.53.

Example 4

(3-Methoxy-5H-dibenzo[a,d]cyclohepten-5-ylidene)acetic acid

A. E-2-[2-(4-Methoxyphenyl)ethenyl]benzonitrile

When diethyl[(2-cyanophenyl)methyl]phosphonate is allowed to react with p-methoxybenzaldehyde in a manner similar to that of Example 1A, the title compound melting at 107.5-108.5°C (uncorr.) is obtained in 97.9% yield. Recrystallisation from cyclohexane (neutral Norit) gives colourless needles melting at 109-110°C (uncorr.) in 89.7% yield.

| | | | |
|---|---|---|---|
| Analysis for: | $C_{16}H_{13}NO$ | | |
| Calculated: | C, 81.68; | H, 5.57; | N, 5.95 |
| Found: | C, 81.39; | H, 5.72; | N, 5.70 |

B. 2-[2-(4-Methoxyphenyl)ethyl]benzonitrile

Reduction of the compound of A above in a manner similar to that of Example 1B, affords the title compound as a crystalline solid melting at 52-56°C (uncorr.) in 98.9% yield. The crude product is purified by distillation, and is obtained as a colourless oil boiling at 147-151.5°C at 0.05mm in a yield of 91.7%. On cooling the distillate crystallizes to a solid melting at 54.56°C (uncorr.)

| | | | |
|---|---|---|---|
| Analysis for: | $C_{16}H_{15}NO$ | | |
| Calculated: | C, 80.98; | H, 6.37; | N, 5.90 |
| Found: | C, 80.73; | H, 6.49; | N, 5.85 |

C. 2-[2-(4-Methoxyphenyl)ethyl]benzoic acid

Hydrolysis of the compound of B above in a manner similar to that of Example 1C gives the title compound as colourless crystals melting at 118.5-119.5°C (uncorr.) in a yield of 95.5%. Recrystallisation from tetrahydrofuran-cyclohexane gives colourless prisms with no change in melting point.

Analysis for: $C_{16}H_{16}O_3$
Calculated: C, 74.98; H, 6.29
Found: C, 74.65; H, 6.25

D. 3-Methoxy-10,11-dihydro-5H-dibenzo[a,d]-cyclohepten-5-one

Polyphosphoric acid (354g) is added to a warm solution of 2-[2-(4-methoxyphenyl)ethyl]benzoic acid (55.0g., 0.217m) in sulfolane (110ml). The mixture is heated to 100-110°C, wherein an exothermic reaction occurs. The pot temperature rises rapidly above 160°C. After the initial reaction subsides, the dark mixture is heated at 90-110° for three hours, poured into distilled water (2.5L) and extracted with methylene chloride (1 x 500ml + 5 x 250ml). The combined extracts are successively washed with water (2 x 250ml), brine (2 x 500ml), filtered through anhydrous sodium sulphate, and the filtrate evaporated in vacuo to give a viscous brown oil. Short-path distillation gives the title compound as an oil boiling at 229°C at 0.08-0.10mm. in a yield of 13.5g (26.1%).

Analysis for: $C_{16}H_{14}O_2$
Calculated: C, 80.60; H, 5.92
Found: C, 81.10; H, 6.04

E. 3-Methoxy-5H-dibenzo[a,d]cyclohepten-5-one

Bromination, dehydrobromination of 3-methoxy-10,11-dihydro-5H-dibenzo[a,d]cyclohepten-5-one in a manner similar to that of Example 1E afford an oily mixture. HPLC (Waters Silica Gel, hexane-methylene chloride 55:45) affords a single peak of pure title compound in 48.3% yield. The structure is confirmed by mass spectrum.

F. (3-Methoxy-5H-dibenzo[a,d]cyclohepten-5-ylidene)acetic acid

The Horner-Wittig condensation of 3-methoxy-5H-dibenzo[a,d]cyclohepten-5-one with triethyl phosphonoacetate is carried out in a manner similar to that of Example 1F. The mixture of E- and Z-isomers of ethyl (3-methoxy-5H-dibenzo[a,d]cyclohepten-5-ylidene)acetate is saponified directly to give, after acidification, the title compound as cream-coloured crystals melting at 182°C dec. (uncorr.) in 85.5% yield. Recrystallisation of the crude product from toluene gives pale yellow prisms melting at 185°C dec. (uncorr.)

| | | |
|---|---|---|
| Analysis for: | $C_{18}H_{14}O_3$ | |
| Calculated: | C, 77.68; | H, 5.07 |
| Found: | C, 77.62; | H, 5.22 |

## Example 5

## (2-Methoxy-5H-dibenzo[a,d]cyclohepten-5-ylidene)acetic acid

A. E-2-[2-(3-Methoxyphenyl)ethenyl]benzonitrile

When diethyl[(2-cyanophenyl)methyl]phosphonate and 3-methoxybenzaldehyde are allowed to react in a manner similar to that of Example 1A, the title compound is obtained as an oil in quantitative yield.

B. 2-[2-(3-Methoxyphenyl)ethyl]benzonitrile

Reduction of the compound of A above in a manner similar to that of Example 1B gives the title compound as a colourless oil boiling at 169-172°C at 0.7-1.0mm in 93.3% yield.

| | | |
|---|---|---|
| Analysis for: | $C_{16}H_{13}NO$ | |
| Calculated: | C, 80.98; | H, 6.37 |
| Found: | C, 80.54; | H, 6.41 |

C. 2-[2-(3-Methoxyphenyl)ethyl]benzoic acid

Hydrolysis of the compound of B above in a manner similar to that of Example 1C followed by acidification affords the title compound as a crystalline solid which upon recrystallization from ethyl acetate melts at 119-121°C.

D. 2-Methoxy-10,11-dihydro-5H-dibenzo[a,d]cyclohepten-5-one

Phosphorus pentachloride (15.4g, 0.0740m) is added to a solution of the compound of C above (17.3g, 0.067m) in benzene (320 ml). The mixture is refluxed for twenty minutes, cooled to 5°C, and a solution of stannic chloride (17.1 ml, 0.146m) in benzene (40ml) is added dropwise with stirring over a period of ninety minutes. The mixture is stirred overnight at ambient temperature. The mixture is cooled to 5° to 10° C (ice bath) and 5N hydrochloric acid added in portions with stirring. After the addition is complete, the organic layer is separated, and the aqueous portion extracted with ether (2X). The combined extracts are successively washed with 1N hydrochloric acid, distilled water (2X), 1N sodium hydroxide (2X), distilled water, brine, then dried over anhydrous magnesium sulphate. The solvents are evaporated *in vacuo* affording a yellow-brown oil which crystallizes on trituration with hexane. The yield of crude ketone is 12.9g (81.0%).

E. 2-Methoxy-5H-dibenzo[a,d]cyclohepten-5-one

Bromination, dehydrobromination of 2-methoxy-10,11-dihydro-5H-dibenzo[a,d]cyclohepten-5-one (26.8g, 0.112m) in a manner similar to that of Example 1E gives a yellow oil which is crystallized from methanol. Purification of the crude product by HPLC gives crystals melting at 75-76°C (uncorr.) in a yield of 9.0g (33.8%).

Analysis for: $C_{16}H_{12}O_2$
Calculated: C, 81.34; H, 5.12
Found: C, 80.90; H, 5.35

F. (2-Methoxy-5H-dibenzo[a,d]cyclohepten-5-ylidene)acetic acid

The Horner-Wittig condensation of 2-methoxy-5H-dibenzo[a,d]cyclohepten-5-one (6.81g, 0.0288m) with triethyl phosphonoacetate in a manner similar to that of Example 1F gives, after saponification of the intermediate E- and Z- esters, the title compound. This is recrystallized from ethyl acetate-hexane to give crystals melting at 195-205°C (uncorr.) in a yield of 4.87g (60.8%). Analytical HPLC indicates a mixture of E- and Z- isomers (82Z:18E).

Analysis for: $C_{18}H_{14}O_3$
Calculated: C, 77.68; H, 5.07
Found: C, 77.74; H, 5.20

## Example 6

## (5H-dibenzo[a,d]cyclohepten-5-ylidene)chloroacetic acid

A. Ethyl(10,11-dihydro-5H-dibenzo[a,d]cycloheptene-5-ylidene)acetate

A solution of triethyl phosphonoacetate (113.0g, 0.504m) in DMSO (100ml) is added dropwise in a nitrogen atmosphere to a magnetically stirred solution of potassium t-butoxide (56.6g, 0.504m) in DMSO (600ml) over a period of ca twenty minutes. The temperature is maintained below 35°C (cold water bath). After stirring at ambient temperature for an additional fifteen minutes, a solution of 10,11-dihydro-5H-dibenzo[a,d]cyclohepten-5-one (95.0g, 0.450m) in DMSO (100ml) is added, and the

mixture heated at 100°C. for twenty-four hours. The excess potassium *t*-butoxide is neutralised with acetic acid (2.9ml) and the mixture concentrated *in vacuo*. The residue is diluted with water (500ml) and extracted with methylene chloride (4 x 250ml). The combined extracts are successively washed with water (2 x 250ml), brine (250ml), filtered through anhydrous sodium sulphate, and concentrated *in vacuo* to give an oil. Distillation of the oil gives the title compound as a pale yellow liquid boiling at 161-169° at 0.5-0.6mm. (189-197° at 1.4-1.6mm) in a yield of 116.8g. (92.0%).

| | | |
|---|---|---|
| Analysis for: | $C_{19}H_{18}O_2$ | |
| Calculated: | C,81.99; | H,6.52 |
| Found: | C,82.06; | H,6.53 |

B. Ethyl(5,*a*-dichloro-10,11-dihydro-5*H*-dibenzo-[*a*,*d*] cyclohepten-5-yl)acetate

A chilled solution of chlorine (6.1g.) in carbon tetrachloride (35ml) is added dropwise to a magnetically stirred solution of ethyl (10,11-dihydro-5*H*-dibenzo[*a*,*d*] cyclohepten-5-ylidene)acetate (20.000g., 0.2186m.) in carbon tetrachloride (125ml) over a period of seven to eight minutes. The temperature is maintained at -20 to -25°C. (dry ice-acetone bath). After stirring at -20 to -25°C for an additional twenty minutes, the dry ice acetone bath is replaced with an ice bath, and the mixture allowed to warm up to ambient temperature overnight. The colourless solid which separates is collected by filtration, washed with carbon tetrachloride, dried at ambient temperature at 0.1mm. The yield of colourless prisms melting at 78° dec. (uncorr.) is 9.793g. (39.0% calculated as title compound).

The filtrate is concentrated *in vacuo* affording an oily solid which is stirred magnetically with pentane (200ml) for two hours. The colourless solid is collected by filtration, washed with pentane, dried at ambient temperature at 0.1mm. The yield of the title compound melting at 90°C. dec. (uncorr.) is 9.721g. (38.73%).

| | | | |
|---|---|---|---|
| Analysis for: | $C_{19}H_{18}Cl_2O_2$ | | |
| Calculated: | C,65.34; | H,5.20; | Cl,20.30 |
| Found: | C,65.32; | H,4.97; | Cl,20.29 |

C. Ethyl(α-chloro-10,11-dihydro-5*H*-dibenzo[*a*,*d*]-cyclohepten-5-ylidene)acetate

A solution of potassium *t*-butoxide (3.086g.) in tetrahydrofuran (50ml) is added dropwise in a nitrogen atmosphere to a magnetically stirred solution of ethyl (5,α-dichloro-10,11-dihydro-5*H*-dibenzo[*a*,*d*]cyclohepten-5-yl)acetate (8.731g.) in tetrahydrofuran (60ml) over a period of about eight minutes while maintaining the temperature below 10°C. (ice-bath). After the addition is complete, the mixture is stirred at ambient temperature for two hours. The mixture is filtered through Celite and the filtrate concentrated *in vacuo* to give an oil which crystallises on cooling. The crude product is diluted with water (50ml) and extracted with methylene chloride (4 x 50ml). The combined extracts are successively washed with water (50ml), brine (50ml), filtered through anhydrous sodium sulphate, then evaporated *in vacuo* to give a colourless oil which crystallises on cooling. The yield of waxy solid melting at 76.5-80.5°C. is 7.477g.(95.61%).

A sample (1.000g) is recrystallised from pentane to give colourless prisms melting at 82.0-84.0°C.(uncorr.)

in a yield of 0.540g (54.0%).

| | |
|---|---|
| Analysis for: | $C_{19}H_{17}ClO_2$ |
| Calculated: | C,72.96; H,5.48; Cl,11.34 |
| Found: | C,72.78; H,5.48; Cl,11.31 |

D. Ethyl(α-chloro-5*H*-dibenzo[*a*,*d*]cyclohepten-5-ylidene)acetate

A mixture of ethyl (α-chloro-10,11-dihydro-5*H*-dibenzo-[*a*,*d*]cyclohepten-5-ylidene)acetate (6.000g.), N-bromosuccinimide (3.448g.) and 2,2'-azobis[2-methylpropionitrile] in dry carbon tetrachloride (100ml) is refluxed for forty-five minutes. After stirring at ambient temperature overnight, 2,2'azobis [2-methyl-propionitrile] (0.250g.) is added, and the mixture refluxed for an additional hour. At this point a negative starch-iodine test is obtained. The succinimide is collected by filtration and washed with carbon tetrachloride (2X). The combined filtrates are evaporated *in vacuo* to give a yellow oil. Triethylamine (50ml) is added to the oil, and the mixture refluxed for two hours. The excess triethylamine is evaporated *in vacuo* affording a solid residue. The crude product is diluted with water (100ml) and extracted with meth-ylene chloride (4 x 100ml). The combined extracts are successively washed with water (2 x 100ml), brine (100ml), filtered through anhydrous sodium sulphate, and the filtrate evaporated *in vacuo* affording an amber oil. HPLC (Water's Silica Gel Column, hexane-methylene chloride 80:20) gives 2.002g (33.58%) of the title compound.

A sample for analysis is recrystallised from pentane and melts at 98.0-100.0°C.(uncorr.)

Analysis for: $C_{19}H_{15}ClO_2$
Calculated: C,73.43; H,4.87; Cl,11.41
Found: C,73.76; H,4.06; Cl,11.25

E. (5H-dibenzo[a,d]cyclohepten-5-ylidene chloroacetic acid

A slurry of ethyl (α-chloro-5H-dibenzo[a,d]cyclohepten-5-ylidene)acetate (1.800g.) in a solution of potassium hydroxide (0.8g.) in a mixture of distilled water (20ml) and 95% ethanol (24ml) is stirred magnetically at ambient temperature for ca sixty hours, then refluxed for one hour. The alcohol is evaporated in vacuo. The aqueous residue is diluted with distilled water (50ml) and extracted with methylene chloride (2 x 50ml). The aqueous layer is added dropwise with stirring to 3N hydrochloric acid. The product separates as a toffee like solid. The acidified mixture is extracted with methylene chloride (3 x 150ml). The combined extracts are successively washed with water (2 x 50ml), brine (50ml), filtered through anhydrous sodium sulphate, and evaporated in vacuo to give a foam. On trituration with pentane, the foam gives a colourless solid melting at 145.5-156.0°C. (uncorr.) Recrystallisation from cyclohexane affords the title compound melting at 153.5-156.0°C. (uncorr.) in a yield of 0.756g. (46.15%)

Analysis for: $C_{17}H_{11}ClO_2$
Calculated: C,72.22; H,3.92; Cl,12.54
Found: C,71.82; H,4.09; Cl,12.17

## Example 7

**5-(5H-Dibenzo[a,d]cyclohepten-5-ylidenemethyl)-1H tetrazole**

A. (5H-Dibenzo[a,d]cyclohepten-5-ylidene)acetonitrile

A solution of diethyl cyanomethylphosphonate (18.980g.) in dimethylsulphoxide (25ml) is added dropwise in a nitrogen atmosphere to a magnetically stirred solution of potassium t-butoxide (11.783g.) in dimethylsulphoxide (125ml) over a period of ca fifteen minutes. After stirring at ambient temperature for an additional five minutes, 5H-dibenzo[a,d]cyclohepten-5-one (21.262g.) is added with the aid of dimethylsulphoxide (50ml). The deep orange solution is heated at 100 ± 1°C. for twenty-four hours. Evaporation of the solvent affords a partially solidified residue which is diluted with water (150ml). The mixture is extracted with methylene chloride (3 x 150ml). The combined extracts are sequentially washed with water (2 x 150ml), brine (150ml), filtered through anhydrous sodium sulphate, and evaporated *in vacuo* to give a tan solid melting at 136.0-139.0°C. (uncorr.) in quantitive yield. Recrystallisation of the crude product from tetrahydrofuran-cyclohexane (Neutral Norit) gives colourless prisms melting at 139.0-140.0°C. (incorr.) in a yield of 17.506g (76.36%).

| | | | |
|---|---|---|---|
| Analysis for: | $C_{17}H_{11}N$ | | |
| Calculated: | C,89.05; | H,4.84; | N,6.11 |
| Found: | C,89.42; | H,5.05; | N,6.24 |

B. 5-(5*H*-Dibenzo[*a*,*d*]cyclohepten-5-ylidenemethyl) 1*H*-tetrazole

A mixture of (5*H*)-dibenzo[*a*,*d*]cyclohepten-5-ylidene)-acetonitrile (7.000g. 0.0305m), sodium azide (2.275g. 0.0350m) and ammonium chloride (1.872g. 0.0350m) in dimethylformamide (100ml) is heated at 130°C. for ten and one half days. The dimethylformamide is evaporated *in vacuo* to give a dark brown solid. This is diluted with water (100ml) and made strongly basic with 50% sodium hydroxide. The aqueous mixture is extracted with methylene chloride (2 x 100ml), then added dropwise with stirring to 6N hydrochloric acid (40ml). The crude product separates as a tan solid which is collected by filtration, washed with water, dried at 100°C./0.1mm. The yield of crystals melting at 211.0°C. dec. (uncorr.) is 25.37%. Recrystallisation from tetrahydrofuran-cyclohexane affords almost colourless crystals (0.860g. 9.02%) melting at 221.0° dec. (uncorr) after drying at 153°C./0.1mm overnight.

| | | | |
|---|---|---|---|
| Analysis for: | $C_{19}H_{12}N_4$ | | |
| Calculated: | C,74.98; | H,4.44; | N,20.58 |
| Found: | C,74.75; | H,4.87; | N,20.46 |

## Example 8

E- and Z-(10-bromo-5*H*-dibenzo[*a*,*d*]cyclohepten-5-ylidene) acetic acid

Triethyl phosphonoacetate (15.559g. 0.0694m) in dimethyl sulphoxide (50ml) is added dropwise in a nitrogen atmosphere to a magnetically stirred suspension of sodium hydride (3.500g. of 50% in oil, 0.0729m) in

dry dimethylsulphoxide (200ml). After stirring at ambient temperature overnight, a solution of 10-bromo-5H-dibenzo-[a,d]cyclohepten-5-one (19.800g. 0.0694m) in dry dimethylsulphoxide (100ml) is added. The mixture is heated at 100 ± 1°C. for 24 hours, then concentrated *in vacuo*. The residue is diluted with a mixture of 50% sodium hydroxide (25ml), distilled water (50ml) and 95% ethanol (75ml). The mixture is refluxed for eighteen hours. The ethanol is evaporated *in vacuo* and the dark oily residue diluted with water (200ml) and extracted sequentially with toluene-heptane (1:1, 100ml) and heptane (100ml). The aqueous layer is treated with Darco G60, filtered, then added dropwise to a magnetically stirred solution of concentrated hydrochloric acid (75ml) in water (100ml). The product separates as a tan solid. The yield of crude product melting at 84-85°C. (uncorr.) is 20.293g. (89.3%) HPLC (Silica Gel, chloroform:hexane: acetic acid 60:40:0.5) gives three fractions. The first fraction (7.28g) is recrystallised from toluene (Darco G60) then from tetrahydrofuran-cyclohexane to give cream coloured crystals melting at 185.0-187.5°C. (uncorr.)

| | | | |
|---|---|---|---|
| Analysis for: | $C_{17}H_{11}BrO_2$ | | |
| Calculated: | C,62.40; | H,3.39; | Br,24.4 |
| Found: | C,62.74; | H,3.42; | Br,24.47 |

## Example 9

### (3-Fluoro-5H-dibenzo[a,d]cyclohepten-5-ylidene acetic acid

A. E-2-[2-(4-fluorophenyl)ethenyl]benzonitrile

The title compound is prepared in a manner similar to

that of Example 1A. The yield of pale yellow crystals melting at 73.0-75.5°C (uncorr.) is 85.0%. Recrystallisation from methylene chloride-pentane (2x) gives colourless prisms melting at 76.0-77.5°C (uncorr.).

Analysis for: $C_{15}H_{10}FN$
Calculated: C,80.70; H,4.52; N,6.27
Found: C,80.48; H,4.62; N,6.25

B. 2-[2-(4-fluorophenyl)ethyl]benzonitrile

The title compound is prepared in a similar manner to that of Example 1B. The yield of product boiling at 130-139°C at 0.4-0.5mm, m.p. 31-33°C (uncorr) is 89.7%.

Analysis for: $C_{15}H_{12}FN$
Calculated: C,79.98; H,5.37; N,6.22
Found: C,79.46; H,5.50; N,6.13

C. 2-[2-(4-fluorophenyl)ethyl]benzoic acid

The title compound is prepared in a manner similar to that of Example 1C. The yield of colourless crystals melting at 134-135.5°C (uncorr) is 92.8%. Recrystallisation from cyclohexane gives colourless needles melting at 134.5-135.0°C (uncorr).

Analysis for: $C_{15}H_{13}FO_2$
Calculated: C,73.76; H,5.36; F,7.78
Found: C,73.22; H,5.38; F,8.00

D. 10,11-dihydro-3-fluoro-5H-dibenzo[a,d]cyclohepten-5-one

The title compound is prepared in a manner similar to that of Example 1D. The yield of pale yellow-orange liquid boiling at 147-155°C at 0.4-0.5mm is 53.4%.

Analysis for: $C_{15}H_{11}FO$

Calculated: C,79.63; H,4.90; F,8.40
Found: C,79.49; H,5.03; F,8.18

E. 3-fluoro-5*H*-dibenzo[*a*,*d*]cyclohepten-5-one

The title compound is prepared in a manner similar to that of Example 1E. HPLC of the crude product on Silica Gel (Waters) gives 57.0% of crystals melting at 107-112°C (uncorr.). Recrystallisation from tetrahydrofuran-cyclohexane (Darco G60)affords colourless crystals melting at 119-120°C (uncorr.).

Analysis for: $C_{15}H_9FO$
Calculated: C,80.35; H,4.05
Found: C,80.04; H,4.18

F. Ethyl (3-fluoro-5*H*-dibenzo[*a*,*d*]cyclohepten-5-ylidene)acetate (mixture of *E*- and *Z*-isomers)

The title compound is prepared in a manner similar to that of Example 1F. The crude mixture of *E*- and *Z*-isomeric esters is obtained in quantitative yield. The ratio of E:Z is approximately 1:1. HPLC separation give the individual *E*- and *Z*-isomers (NMR spectra).

Recrystallisation of the *Z*-isomer from pentane gives colourless needles melting at 84.0-85.0°C (uncorr.).

Analysis for: $C_{19}H_{15}FO_2$
Calculated: C,77.53; H,5.14; F,6.46
Found: C,77.30; H,5.11; F,6.35

Recrystallisation of the *E*-isomer from pentane affords colourless crystals melting at 60-62°C (uncorr.).

| | |
|---|---|
| Analysis for: | $C_{19}H_{15}FO_2$ |
| Calculated: | C,77.53; H,5.14; F,6.46 |
| Found: | C,77.40; H,5.13; F,6.29 |

G. Z-(3-fluoro-5H-dibenzo[a,d]cyclohepten-5-ylidene acetic acid

The title compound is prepared in a manner similar to that of Example 1G. The yield of Z-acid melting at 221.5°C dec. (uncorr.) is 98.6%. Recrystallisation from tetrahydrofurancyclohexane (Darco G60) gives colourless prisms melting at 228.0°C dec. (uncorr.).

| | |
|---|---|
| Analysis for: | $C_{17}H_{11}FO_2$ |
| Calculated: | C,76.68; H,4.16 |
| Found: | C,77.00; H,4.16 |

H. E-(3-Fluoro-5H-dibenzo[a,d]cyclohepten-5-ylidene acetic acid

The title compound is prepared in a manner similar to that of Example 1H. The yield of E-acid melting at 187.5°C dec. (uncorr.) is 87.6%. Recrystallisation from tetrahydrofuran-cyclohexane(Darco G60) affords pale yellow prisms melting at 190°C dec. (uncorr.).

| | |
|---|---|
| Analysis for: | $C_{17}H_{11}FO_2$ |
| Calculated: | C,76.68; H,4.16 |
| Found: | C,77.10; H,4.36 |

## Example 10

The anti-inflammatory activity of the compounds of the invention is assessed by their ability to inhibit experimentally induced edema in the hind paw of the rat, which is an acute inflammatory response.

Groups of 6-8 male Sprague-Dawley rats weighing between 150-200 grams are used. Compounds of the invention, aspirin and indomethacin are administered orally in 0.5% methylcellulose. Vehicle alone is administered as a control. Sixty minutes after drug administration edema is induced by injection of 0.1 ml. of a 1% carrageenan solution in saline into the subplantar tissue of the rat's right hind paw. Two minutes later, paw volume is measured volumetrically with a plethysmograph and again 3 hours later. The mean volume of swelling for the control group is calculated and compared to the test groups. Percent change in paw edema is calculated and analysed statistically (unpaired, Student's t-test).

The results are summarised in Table 1

Table 1

| Compound of Example No. | Oral Dose mg/kg | % Change From Control |
|---|---|---|
| 1G. (Z-isomer) | 100-200 | -20 |
| 1H. (E-isomer) | 20 | -33 |
| | 60 | -53 |
| | 180 | -68 |
| 2G. (Z-isomer) | 200 | ~ -38 |
| 2H. (E-isomer) | 200 | -49 |
| 4F. (mixture of isomers) | >200 | -50 |
| 6E. | 200 | -31 |
| 7B. | 200 | ~ -38 |
| 8. (mixture of isomers) | 200 | -34 |
| 9. (mixture of isomers) | 200 | -58 |

Indomethacin, orally dosed, exhibits a 50% inhibition of edema at a dose of about 5.6 mg/kg, and aspirin exhibits a 50% inhibition of edema at a dose of about 250 mg/kg.

The results show that E-(3-chloro-5H-dibenzo[a,d] cyclohepten-5-ylidene acetic acid and (3-fluoro-5H-dibenzo[a,d]cyclohepten-5-ylidene)acetic acid (mixture of isomers) inhibit carrageenan paw edema at oral doses of 20-200 mg/kg, which is a more significant reduction in the acute inflammatory response than that attained by aspirin.

Example 11

The ability of the compounds of the invention to inhibit immunologic-induced (chronic) inflammation is tested in adjuvant arthritis (daily dosing) assay.

Polyarthritis is induced in groups of 6-10 Sprague-Dawley rats weighing between 180-220 g. by a subcutaneous injection into the right paw of dessicated Mycobacterium butyricum or tuberculosis (0.5 mg/0.1 ml) suspended in light mineral oil. Compounds of the invention, aspirin and indomethacin are administered orally in 0.5% methylcellulose using a daily regimen (except for weekends). Both hind paw volumes (ml.) are measured by mercury plethysmograph on day 0 or at the time of injection of the complete adjuvant. Increases in paw volume (viz. edema) are determined for the left paw on day 16. Statistical analysis of differences in paw edemas is performed by using the unpaired, Student's t-test.

The results are summarised in Table 2.

Table 2

| Compound of Example No. | Oral Dose mg/kg | % Change of Left Paw Edema at Day 16 |
|---|---|---|
| 1G. (Z-isomer) | 25 | -28 |
| 1H. (E-isomer) | 25 | -59 |
| 2H. (E-isomer) | 25 | -59 |
| 4F. (mixture of isomers) | 15 | -50 |
| 8. (mixture of isomers) | 25-50 | -25 to -50 |
| 9. (mixture of isomers) | 100-200 | -40 |

The results show that the compounds of the invention are effective at doses of about 25-200 mg/kg. (orally) using left paw edema (i.e. immunologically induced inflammation) as the major parameter. The estimated $ED_{50}$ of indomethacin in this test is 0.9 mg/kg. and that of aspirin has been assessed at approximately 140 mg/kg. Thus, the compounds of the invention show significant anti-inflammatory activity in the adjuvent arthritis test.

CLAIMS

1. A compound having the formula:

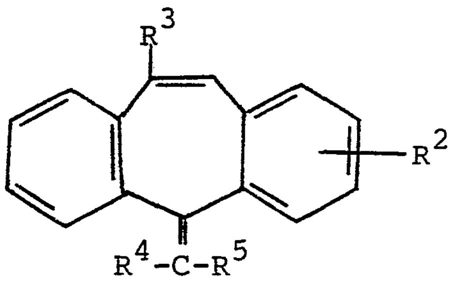

B

wherein $R^4$ is hydrogen or chloro;
$R^5$ is carboxy, an ester thereof, or tetrazolyl;
$R^2$ is hydrogen, halo, or alkoxy of 1 to 4 carbon atoms and
$R^3$ is hydrogen or bromo,
with the proviso that when $R^4$ is hydrogen and $R^5$ is carboxy then $R^2$ or $R^3$ is other than hydrogen; and that when $R^4$, $R^2$ and $R^3$ are all hydrogen, $R^5$ is tetrazolyl; the stereoisomers thereof and the pharmaceutically acceptable salts thereof.

2. (3-Chloro-5*H*-dibenzo[*a*,*d*]cyclohepten-5-ylidene) acetic acid, (2-Chloro-5*H*-dibenzo[*a*,*d*]cyclohepten-5-ylidene) acetic acid, or (3-Fluoro-5*H*-dibenzo[*a*,*d*]cyclohepten-5-ylidene) acetic acid, or a pharmaceutically acceptable salt thereof.

3. (3-Methoxy-5*H*-dibenzo[*a*,*d*]cyclohepten-5-ylidene) acetic acid or a pharmaceutically acceptable salt thereof.

4. (5*H*-Dibenzo[*a*,*d*]cyclohepten-5-ylidene) chloroacetic acid or a pharmaceutically acceptable salt thereof.

5. 5-(5*H*-Dibenzo[*a*,*d*]cyclohepten-5-ylidenemethyl)-1*H*-tetrazole or a pharmaceutically acceptable salt thereof.

6. (10-Bromo-5H-dibenzo[a,d]cyclohepten-5-ylidene) acetic acid or a pharmaceutically acceptable salt thereof.

7. The E stereoisomers of a compound of formula B as claimed in Claim 1, wherein $R^2$ is halo or alkoxy of 1-4 carbon atoms or a pharmaceutically acceptable salt thereof.

8. E-(3-Chloro-5H-dibenzo[a,d]cyclohepten-5-ylidene) acetic acid or a pharmaceutically acceptable salt thereof.

9. E-(2-Chloro-5H-dibenzo[a,d]cyclohepten-5-ylidene) acetic acid or a pharmaceutically acceptable salt thereof.

10. A pharmaceutical composition comprising a compound of Formula B, as claimed in Claim 1, or Claim 7, wherein $R^5$ is carboxy or tetrazolyl and $R^2$, $R^3$ and $R^4$ are as defined in Claim 1, or a pharmaceutically acceptable salt thereof and a pharmaceutically acceptable carrier.

11. A pharmaceutical composition comprising a compound as claimed in any one of claims 2 to 6, 8 or 9 and a pharmaceutically acceptable carrier.

12. A pharmaceutical composition as claimed in Claim 10 or Claim 11 in unit dosage form.

13. A compound of formula B, as claimed in Claim 1, or Claim 7, wherein $R^5$ is carboxy or tetrazolyl and $R^2$, $R^3$ and $R^4$ are as defined in Claim 1, or a compound as claimed in any one of Claims 2 to 6, 8 or 9, or a pharmaceutically acceptable salt thereof, for use as a pharmaceutical.

14. A compound of formula B, as claimed in Claim 1 or Claim 7, wherein $R^5$ is carboxy or tetrazolyl and $R^2$, $R^3$ and $R^4$ are as defined in Claim 1, or a compound as claimed in any one of claims 2 to 6, 8 or 9 or a pharmaceutically acceptable salt thereof, for use as an anti-inflammatory agent.

15. A method for preparing a compound of Formula B as claimed in Claim 1 wherein $R^5$ is carboxy or a pharmaceutically acceptable salt thereof characterised in that a compound of formula B, wherein $R^5$ is a carboxylic ester is hydrolysed to obtain a compound of Formula B, wherein $R^5$ is carboxy or a pharmaceutically acceptable salt thereof.

16. A method for preparing a compound of Formula B as claimed in Claim 1, wherein $R^5$ is tetrazolyl or a pharmaceutically acceptable salt thereof, characterised in that a compound of Formula C

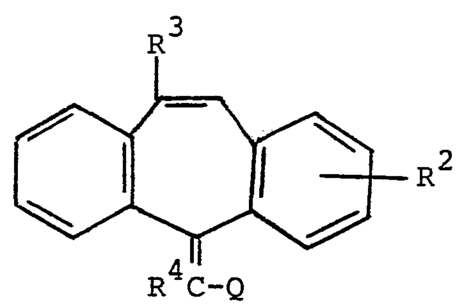

C

wherein $R^4$, $R^2$ and $R^3$ are as defined in Claim 1, and Q is a group containing an unsaturated -C-N linkage, is reacted with other known compounds to form a tetrazole ring system and thus a product of Formula B and if desired converting the product to a pharmaceutically acceptable salt thereof.

CLAIMS - AUSTRIA

1. A process for preparing a compound of Formula B

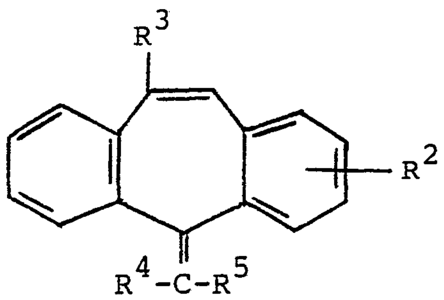

B

wherein $R^4$ is hydrogen or chloro;
$R^5$ is carboxy, an ester thereof, or tetrazolyl;
$R^2$ is hydrogen, halo or alkoxy of 1 to 4 carbon atoms and
$R^3$ is hydrogen or bromo,

with the proviso that when $R^4$ is hydrogen and $R^5$ is carboxy then $R^2$ or $R^3$ is other than hydrogen; and that when $R^4$, $R^2$ and $R^3$ are all hydrogen, $R^5$ is tetrazolyl; the stereoisomers thereof and the pharmaceutically acceptable salts thereof characterised in that a compound of Formula D

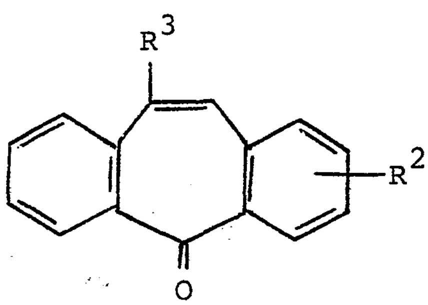

D

wherein $R^2$ and $R^3$ are as defined above is treated in known manner to introduce the group $R^4-\overset{\|}{C}-R^5$ wherein $R^4$ and $R^5$ are as defined above and if desired the product is converted to a pharmaceutically acceptable salt.

2. A process for preparing a compound of Formula B

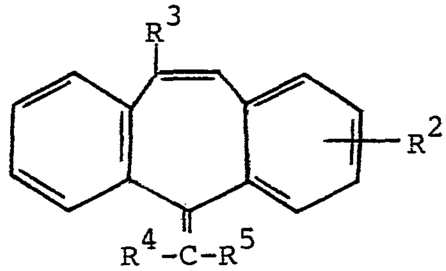

B

wherein $R^5$ is carboxy and $R^2$, $R^3$ and $R^4$ are as defined in Claim 1, or a pharmaceutically acceptable salt thereof, characterised in that a compound of Formula B wherein $R^5$ is a carboxylic ester is hydrolysed.

3. A process for preparing a compound of formula B

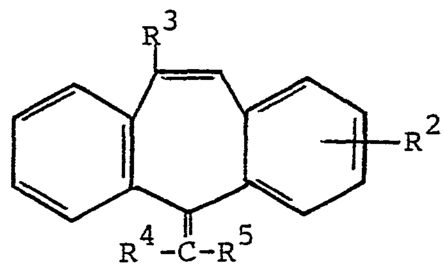

B

wherein $R^5$ is tetrazolyl or a pharmaceutically acceptable salt thereof, and $R^2$, $R^3$ and $R^4$ are as defined in Claim 1 characterised in that a compound of Formula C

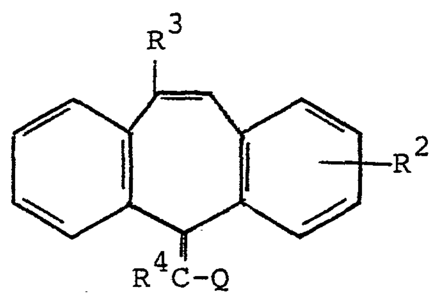

C

wherein $R^2$, $R^3$ and $R^4$ are as defined above and Q is a group containing an unsaturated -C-N linkage, is reacted

with other known compounds to form a tetrazole ring system and thus a product B wherein $R^5$ is tetrazolyl and if desired the product is converted to a pharmaceutically acceptable salt.

4. A process as claimed in Claim 3 for preparing a compound of Formula B characterised in that a compound of formula C as defined in Claim 3 wherein Q is $-C{\equiv}N$ or $-\underset{G}{\overset{|}{C}}{=}NH$ wherein G is a halogen atom or an alkoxy radical is reacted with an alkali metal azide or ammonium azide to obtain a compound of formula B wherein $R^5$ is tetrazolyl and if desired the product is converted to a pharmaceutically acceptable salt.

5. A process as claimed in Claim 2, characterised in that the E isomer of a compound of Formula B wherein $R^2$ is halo or alkoxy of 1 to 4 carbon atoms and $R^5$ is a carboxylic ester is hydrolysed.

6. A process for preparing an anti-inflammatory composition characterised in that a compound of Formula B, as defined in Claim 1, wherein $R^5$ is carboxy or tetrazolyl and $R^2$, $R^3$ and $R^4$ are as defined in Claim 1 or a pharmaceutically acceptable salt thereof is mixed with a pharmaceutical carrier and formulated into an anti-inflammatory preparation.

7. A process as claimed in Claim 6, characterised in that the composition is formulated into unit dosage forms such as tablets, capsules and the like.

8. A process as claimed in Claim 6, or Claim 7, characterised in that a compound of Formula B, as defined in

Claim 6, or a pharmaceutically acceptable salt thereof, is prepared by a method known for analogous compounds and then mixed with a pharmaceutically acceptable carrier and formulated into an anti-inflammatory preparation.

9. A process as claimed in Claim 8, characterised in that the compound of Formula B is prepared by a process as claimed in any one of claims 1-5.

10. A process for preparing an anti-inflammatory composition characterised in that a compound of Formula B as defined in Claim 1 wherein $R^5$ is carboxy or tetrazolyl and $R^2$, $R^3$ and $R^4$ are as defined in Claim 1 is treated with a base to obtain a salt thereof and the salt is mixed with a pharmaceutically acceptable carrier and formulated into anti-inflammatory preparations.

European Patent Office

# EUROPEAN SEARCH REPORT

0115690

Application number

EP 83 30 7839

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A | THE JOURNAL OF ORGANIC CHEMISTRY, vol. 37, no. 26, 29th December 1972, pages 4294-4302 G.N. WALKER et al.: "Synthesis of 10,11-dihydro-5,10-ethano-5H-dibenzo[a,d]cycloheptenes with various side chains at position 12" * Page 4299, column 1, lines 40-54; column 2, lines 3-15 * | 1 | C 07 C 57/62<br>C 07 C 59/72<br>C 07 C 69/618<br>A 61 K 31/19<br>A 61 K 31/215<br>A 61 K 31/41<br>C 07 D 257/04 |
| A | GB-A-1 053 844 (N.V. KONINKLIJKE PHARMACEUTISCHE FABRIEKEN) * Claim 1; page 1, lines 23-25; page 2, figure V * | 1,10 | |
| A | BE-A- 633 316 (MERCK & CO.) * Claim 1 * | 1 | **TECHNICAL FIELDS SEARCHED (Int. Cl. 3)** |
| | | | C 07 C 57/00<br>C 07 C 59/00<br>C 07 C 69/00 |

The present search report has been drawn up for all claims

| Place of search | Date of completion of the search | Examiner |
|---|---|---|
| THE HAGUE | 09-04-1984 | KLAG M.J. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document